(19) Europäisches Patentamt
European Patent Office
Office européen des brevets

(11) EP 0 736 532 B1

(12) **EUROPÄISCHE PATENTSCHRIFT**

(45) Veröffentlichungstag und Bekanntmachung des Hinweises auf die Patenterteilung:
**15.05.2002 Patentblatt 2002/20**

(51) Int Cl.$^7$: **C07D 471/14**, A61K 31/495

(21) Anmeldenummer: **96103855.1**

(22) Anmeldetag: **12.03.1996**

(54) **Pyrido(3,2-e)pyrazinone mit antiasthmatischer Wirksamkeit und Verfahren zu deren Herstellung**

Pyrido(3,2-e)pyrazinones with antiasthmatic activity and process for their preparation

Pyrido(3,2-e)pyrazinones possédant une activité anti-asthmatique et procédé de préparation

(84) Benannte Vertragsstaaten:
**AT BE CH DE DK ES FI FR GB GR IE IT LI LU MC NL PT SE**
Benannte Erstreckungsstaaten:
**SI**

(30) Priorität: **24.03.1995 DE 19510965**

(43) Veröffentlichungstag der Anmeldung:
**09.10.1996 Patentblatt 1996/41**

(73) Patentinhaber: **ASTA Medica Aktiengesellschaft**
**01277 Dresden (DE)**

(72) Erfinder:
- **Höfgen, Norbert, Dr.**
  **01129 Dresden (DE)**
- **Büchner, Thomas, Dr.**
  **53121 Bonn (DE)**
- **Achterrath-Tuckermann, Ute, Dr.**
  **63477 Maintal (DE)**
- **Szelenyi, Stefan, Prof.**
  **90571 Schwaig (DE)**
- **Kutscher, Bernhard, Prof.**
  **63477 Maintal (DE)**

(74) Vertreter: **Dey, Michael, Dr.**
**Weickmann & Weickmann,**
**Patentanwälte,**
**Postfach 86 08 20**
**81635 München (DE)**

(56) Entgegenhaltungen:
**EP-A- 0 400 583** **EP-A- 0 584 487**

- **J. MED. CHEM., Bd. 34, Nr. 9, 1991, Seiten 2671-7, XP002007870 DAVEY, D.D.; ERHARDT, P.W.; CANTOR, E.H.; GREENBERG, S.S.; INGEBRETSEN, W.R.; WIGGINS, J.: "Novel Compounds Possessing Potent cAMP and cGMP Phosphodiesterase Inhibitory Activity. Synthesis and Cardiovascular Effects of a Series of Imidazo[1,2-a]quinoxalinones and Imidazo[1,5-a]quinoxalinones and Their Aza Analogues"**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 736 532 B1

Printed by Jouve, 75001 PARIS (FR)

## Beschreibung

Die Erfindung betrifft Pyrido [3,2-e]pyrazinone.

Die Europa - Patentanmeldung 0 400 583 betrifft Imidochinoxaline und deren Aza - Analoge der allgemeinen Formel

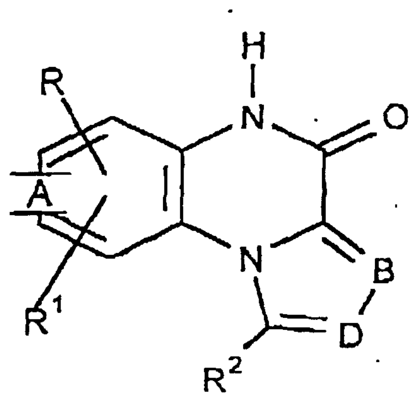

worin A ein Stickstoffatorn oder CH, B und D ein Stickstoffatom oder CH beziehungswelse ein substituiertes Kohlenstoffatom bedeuten und die Reste R, $R^1$, $R^2$ Wasserstoff oder verschiedene organische Substituenten darstellen. Für diese Verbindungen wird eine positiv inotrope gefäßerweiternde Wirkung angegeben.

Weiterhin wird im Indian Journal of Chemistry, Band 10, 1972, Seiten 344 - 350 unter anderem die Herstellung von Verbindungen der Formel

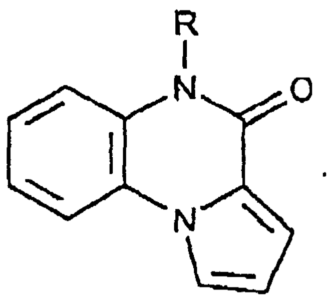

R = $-(CH_2)_nNR^1R^2$

beschrieben , worin der Rest R 3-Dimethylaminopropyl-(1), 2-Morpholinoethyl-(1), 2-Pyrrolidinoethyl-(1) oder 2-Dimethylaminoethyl-(1) sein kann. Eine pharmakologische Wirkung wird nicht angegeben .

Die Europa - Patentanmeldung 0 584 487 betrifft 4,5-Dihydro-4-oxo-pyrrolo[1,2-a]-chinoxaline und deren Azaanaloge der allgemeinen Formel

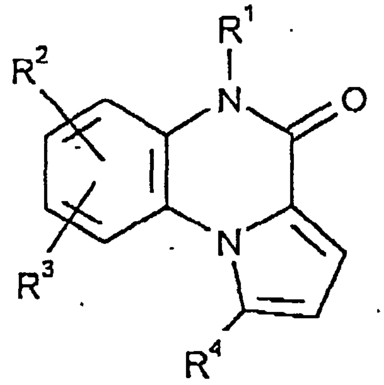

wobei die Reste $R^1$, $R^2$, $R^3$ und $R^4$ für eine Vielzahl organischer Substituenten stehen.

Für diese Verbindungen werden antiallergische, antiasthmatische, anxiolytische, hypotensive und vasodilatierende Wirkungen sowie eine positiv inotrope Wirkung beschrieben die ursächlich auf einer selektiven PDE III - Hemmung beruhen.

Die Patentanmeldung WO(PCT) 93 20 077 bezieht sich auf Imidazochinoxalinone der allgemeinen Formel

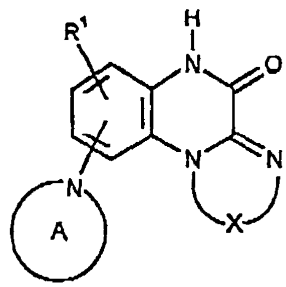

wobei A für 5-Ring-Heterocyclen mit 2 oder 3 Stickstoffatomen im Ring steht, $R^1$ $NO_2$ oder $CF_3$ sein kann und X für verschiedene, zum Teil Stickstoff enthaltende Ketten mit bis zu 4 Kettengliedem steht.

Diese Verbindungen werden als Glutamat - Rezeptor Antagonisten mit psychotroper sowie antiischämischer Wirkung beschrieben.

Die japanischen Patentanmeldungen JP 06 128 261 und JP 06 128 262 beziehen sich auf die Herstellung von Verbindungen der allgemeinen Formel

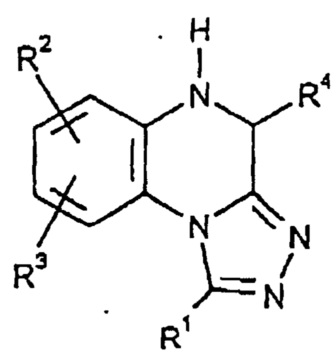

wobei die Reste $R^1$, $R^2$, $R^3$ und $R^4$ für verschiedene organische Substituenten stehen. Eine pharmakologische Wirkung wird nicht angegeben.

Die Europa - Patentanmeldung 0 623 620 betrifft die Herstellung von Verbindungen der allgemeinen Formel

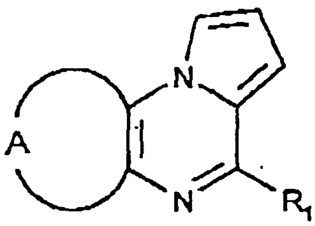

wobei A für anellierte aromatische oder heteroaromatische Ringsysteme und $R_1$ für substituierte Amino-Gruppen stehen. Die beschriebenen Verbindungen besitzen teilweise $5HT_3$ - agonistische Wirkungen.

Die Europa - Patentanmeldung 0 518 530 bezieht sich auf die Herstellung von Verbindungen der allgemeinen Formeln

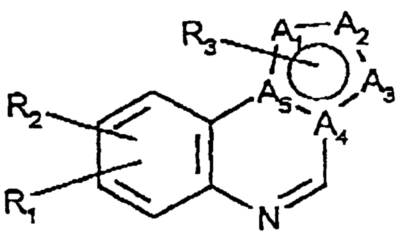

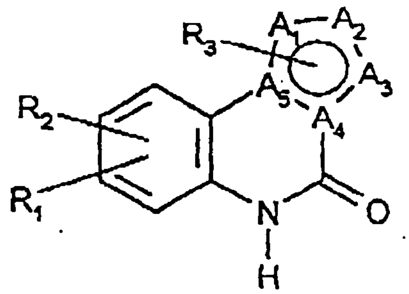

wobei $R_1$, $R_2$ und $R_3$ für verschiedene organische Substituenten stehen und $A_1$ bis $A_5$ für C oder N stehen, wobei mindestens zwei von ihnen N bedeuten. Diese Verbindungen sind Antagonisten von Rezeptoren excitatorischer Aminosäuren.

Die Offenlegungsschrift DE 43 29 970 betrifft die Herstellung von Verbindungen der allgemeinen Formel

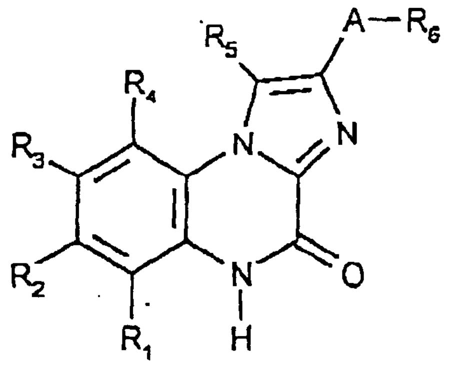

wobei A für eine gesättigte oder ungesättigte Alkylen-Gruppe mit 1-5 C-Atomen steht, $R_1$, $R_2$, $R_3$, $R_4$ and $R_5$ verschiedene organische Substituenten bedeuten und $R_6$ für eine funktionelle Gruppe steht, die eine Carbonylgruppe enthält. Diese Verbindungen werden als Antagonisten von Rezeptoren excitatorischer Aminosäuren beschrieben.

Die Erfindung betrifft die durch die Patentansprüche angegebenen Verbindungen. Die erfindungsgemäßen neuen Verbindungen sind pharmakologisch wirksam und besitzen insbesondere starke antiasthmatische und antiallergische Wirkungen auf der Basis selektiver PDE IV/V - Hemmung.

Der Erfindung liegt die Aufgabe zugrunde, neue Verbindungen mit wertvollen pharmakologischen Eigenschaften zur Verfügung zu stellen.

Die Erfindung betrifft darüber hinaus die Verfahren zur Herstellung der in den Patentansprüchen angeführten neuen Verbindungen sowie zu ihrer Anwendung .

Weiterhin betrifft die Erfindung die physiologisch verträglichen Salze der Verbindungen gemäß Formel I, die Verfahren zur Herstellung der Verbindungen gemäß Formel I und ihre pharmazeutische Verwendung.

Die Erfindung betrifft Pyrido[3,2-e]pyrazinone der Formel

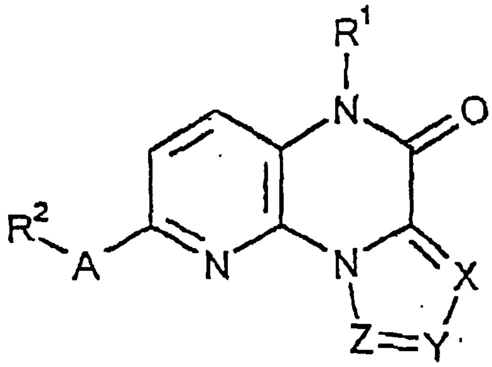

I

worin

A Sauerstoff bedeutet;
X, Y und Z für N oder $CR^3$ stehen, wobei mindestens einer von X, Y und Z N bedeuten muss;

$R^1$ $C_1$-$C_{10}$-Alkyl (gegebenenfalls auch verzweigt), das ein- oder mehrfach substituiert sein kann mit Hydroxy-, $C_1$-$C_6$-Alkyloxy-, Alkenyloxy- mit bis 6 C-Atomen, Alkinyloxy- mit bis 6 C-Atomen, Aryl-, Aryloxy-, Heteroaryl-, Heteroaryloxy-, Amino-, Halogen, $NO_2$, CN, C=$OR^4$ oder $S(O)_nR^5$ (mit n von 0 bis 2); Alkenyl mit bis 10 C-Atomen (gegebenenfalls auch verzweigt), das ein- oder mehrfach substituiert sein kann mit Hydroxy-, $C_1$-$C_6$-Alkyloxy-, Alkenyloxy- mit bis 6 C-Atomen, Alkinyloxy- mit bis 6 C-Atomen, Aryl-, Aryloxy-, Heteroaryl-, Heteroaryloxy-, Amino, Halogen, $NO_2$, CN, C = $OR^4$ oder $S(O)_nR^5$ (mit n von 0 bis 2); oder $C_5$-$C_7$-Cycloalkyl, das ein- oder mehrfach substituiert sein kann mit Hydroxy-, $C_1$-$C_6$-Alkyloxy-, Alkenyloxy- mit bis zu 6 C-Atomen, Alkinyloxy- mit bis 6 C-Atomen, Aryl-, Aryloxy-, Heteroaryl- Heteroaryloxy-, Amino-, Halogen, $NO_2$, CN, C = $OR^4$ oder $S(O)_nR^5$ (mit n von 0 bis 2) darstellt;

$R^2$ für H; $C_1$-$C_{10}$-Alkyl (gegebenenfalls auch verzweigt), das einoder mehrfach substituiert sein kann mit Hydroxy-, $C_1$-$C_6$-Alkyloxy, Alkenyloxy- mit bis 6 C-Atomen, Alkinyloxy- mit bis 6 C-Atomen, Aryl-, Aryloxy-, Heteroaryl-, Heteroaryloxy-, Amino-, Halogen, $NO_2$, CN, C=$OR^4$ oder $S(O)_nR^5$ (mit n von 0 bis 3); Alkenyl mit bis 10 C-Atomen (gegebenenfalls auch verzweigt), das ein- oder mehrfach substituiert sein kann mit Hydroxy-, $C_1$-$C_6$-Alkyloxy-, Alkenyloxy- mit bis 6 C-Atomen, Alkinyloxy- mit bis 6 C-Atomen; Aryl-, Aryloxy-, Heteroaryl-, Heteroaryloxy-, Amino-, Halogen, $NO_2$, CN, C = $OR^4$ oder $S(O)_nR^5$ (mit n von 0 bis 2); oder $C_5$-$C_7$-Cycloalkyl, das ein- oder mehrfach substituiert sein kann mit Hydroxy-, $C_1$-$C_6$-Alkyloxy-, Alkenyloxy- mit bis 6 C-Atomen, Alkinyloxy- mit bis 6 C-Atomen, Aryl-, Aryloxy-, Heteroaryl-, Heteroaryloxy-, Amino-, Halogen, $NO_2$, CN, C=$OR^4$ oder $S(O)_nR^5$ (mit n von 0 bis 2) stehen kann;

$R^3$ H; $C_1$-$C_6$-Alkyl (gegebenenfalls verzweigt) bedeutet;

$R^4$ für H; $C_1$-$C_6$-Alkyl (gegebenenfalls verzweigt); Phenyl; OH; $C_1$-$C_6$-Alkyloxy (gegebenenfalls verzweigt); Aryloxy; Amino steht;

$R^5$ H; $C_1$-$C_6$-Alkyl; Aryl; OH; $C_1$-$C_6$-Alkytoxy; Aryloxy; Amino bedeutet und ihre physiologisch verträglichen Salze sowie Verbindungen worin

| X | Y | Z | A | R1 | R2 |
|---|---|---|---|---|---|
| $C-CH_3$ | N | $C-C_2H_5$ | O | $CH_2C_6H_4$(2-Cl) | $CH_3$ |
| $C-CH_3$ | N | $C-C_2H_5$ | O | $CH_2C_6H_4$(4-Cl) | $CH_3$ |
| $C-CH_3$ | N | $C-C_2H_5$ | O | $CH_2C_6H_3$(2,4-di-Cl) | $CH_3$ |
| $C-CH_3$ | N | $C-C_2H_5$ | O | $CH_2C_6H_3$(2,6-di-Cl) | $CH_3$ |
| $C-CH_3$ | N | $C-C_2H_5$ | O | $CH_2C_6H_4$(2-F) | $CH_3$ |
| $C-CH_3$ | N | $C-C_2H_5$ | O | $CH_2C_6H_4$(4-F) | $CH_3$ |
| $C-CH_3$ | N | $C-C_2H_5$ | O | $CH_2C_6H_3$(2-Cl,6-F) | $CH_3$ |
| $C-CH_3$ | N | $C-C_2H_5$ | O | $CH_2C_6H_4(2-CH_3)$ | $CH_3$ |
| $C-CH_3$ | N | $C-C_2H_5$ | O | $CH_2C_6H_4(4-OCH_3)$ | $CH_3$ |
| $C-CH_3$ | N | $C-C_2H_5$ | O | $CH_2C_6H_2(3,4,5-tri-OCH_3)$ | $CH_3$ |
| $C-CH_3$ | N | $C-C_2H_5$ | O | $CH_2C_6H_4(4-OCH_2-C_6H_5)$ | $CH_3$ |
| $C-CH_3$ | N | $C-C_2H_5$ | O | $C_2H_5$ | $(CH_2)_3CON(CH_3)C_6H_{11}$ |
| $C-CH_3$ | N | $C-C_2H_5$ | O | $CH_3$ | $CH_2C_6H_4$(4-F) |

| X | Y | Z | A | $R^1$ | $R^2$ |
|---|---|---|---|---|---|
| $C-CH_3$ | N | $C-C_2H_5$ | O | $C_2H_5$ | $CH_2C_6H_4(4-F)$ |
| $C-CH_3$ | N | $C-C_2H_5$ | O | $CH_2C_6H_2(3,4,5-tri-OCH_3)$ | $CH_2C_6H_2(3,4,5-tri-OCH_3)$ |
| $C-CH_3$ | N | $C-C_2H_5$ | O | $C_2H_5$ | 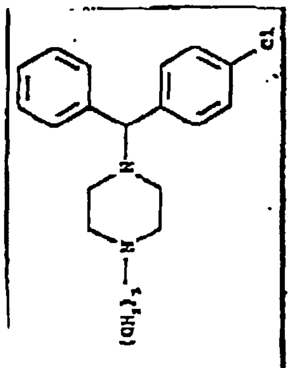 |

Die Erfindung betrifft weiterhin ein Verfahren zur Herstellung Pyrido[3,2-e]pyrazinone der Formel I, worin A, X, Y, Z, $R^1$, $R^2$, $R^3$, $R^4$ und $R^5$ die im Anspruch 1 genannte Bedeutung haben, welches dadurch gekennzeichnet ist, daß Verbindungen der Formel

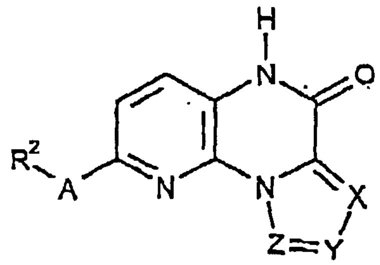

II

worin X, Y , Z, A und $R^2$ die im Anspruch 1 angeführte Bedeutung haben , mit $R^1$ - Hal (Hal = Halogen) in Gegenwart eines anorganischen oder organischen basischen Katalysators umgesetzt werden, wobei $R^1$ die im Anspruch 1 ausgeführte Bedeutung basifzt.

Ein weiterer Anspruch der Erfindung ist ein Verfahren zur Herstellung Pyrido[3,2-e]pyrazinone der Formel I, worin A, X, Y, Z, $R^1$, $R^2$, $R^3$, $R^4$ und $R^5$ die im Anspruch 1 genannte Bedeutung haben, welches dadurch gekennzeichnet ist, daß Verbindungen der Formel

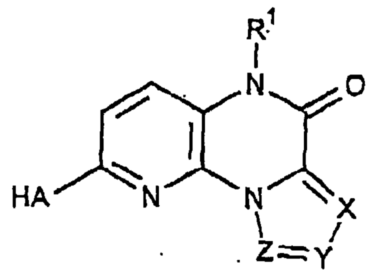

III

worin A, X, Y, Z und $R^1$ die im Anspruch 1 angeführte Bedeutung haben, mit $R^2$ - Hal (Hal = Halogen) in Gegenwart eines anorganischen oder organischen basischen Katalysators umgesetzt werden, wobei $R^2$ die im Anspruch 1 ausgeführte Bedeutung besitzt.

Bevorzugt überführt man beim erfindungsgemäßen Verfahren basische Verbindungen der Formel I in Salze.

Die Verbindungen der Formel I nach Anspruch 1 eignen sich zur Anwendung als therapeutische Wirkstoffe mit antiasthmatischen und antiallergischen Wirkungen .

Ein weiterer Anspruch der Erfindung ist ein Arzneimittel, enthaltend eine oder mehrere Verbindung der Formel I nach Anspruch 1 neben üblichen physiologisch verträglichen Trägern und/oder Verdünnungsmitteln beziehungsweise Hilfsstoffen.

Die Erfindung betrifft auch ein Verfahren zur Herstellung eines Arzneimittels nach Anspruch 7, welches dadurch gekennzeichnet ist, daß eine oder mehrere Verbindung der Formel I mit gebräuchlichen pharmazeutischen Trägerstoffen und/oder Verdünnungsmitteln beziehungsweise sonstigen Hilfsstoffen zu pharmazeutischen Zubereitungen verarbeitet beziehungswelse In eine therapeutisch anwendbare Form gebracht werden sowie die Verwendung von Verbindungen der allgemeinen Formel I nach Anspruch 1 zur Herstellung von Arzneimitteln nach den Ansprüchen 7 und 8 zur Anwendung bei asthmatischen und allergischen Erkrankungen allein oder in Kombination untereinander oder in Kombination mit Trägerstoffen und/oder Verdünnungsmitteln beziehungsweise sonstigen Hilfsstoffen.

Diejenigen Verbindungen der Formel I, die asymmetrische Kohlenstoffatome enthalten und in der Regel als Razemate anfallen, können in an sich bekannter Weise beispielsweise mit einer optisch aktiven Säure in die optisch aktiven

Isomeren gefrennt werden . Es ist aber auch möglich, von vornherein eine optisch aktive Ausgangssubstanz einzusetzen, wobei dann als Endprodukt eine entsprechende optisch aktive beziehungsweise diastereomere Verbindung erhalten wird. Die Erfindung umfaßt also von Verbindungen der Formel I, die ein asymmetrisches Kohlenstoffatom enthalten, die D-Form, die L-Form und D.L-Mischungen sowie im Falle mehrerer asymmetrischer Kohlenstoffatome die diastereomeren Formen.

Je nach Verfahrensbedingungen und Ausgangsstoffen können die Verbindungen der Formel I als freie Verbindungen oder in Form ihrer Salze erhalten werden Die erhaltenen Salze können in an sich bekannter Weise beispielsweise mit Alkali oder Ionenaustauschem in die freien Basen beziehungsweise mit anorganischen oder organischen Säuren in die freien Säuren überführt werden. Von den so freigesetzten Verbindungen der Formel I lassen sich

durch Umsetzung mit anorganischen oder organischen Säuren beziehungsweise mit anorganischen oder organischen Basen , die zur Bildung therapeutisch verwendbarer Salze geeignet sind , Salze gewinnen .

Die erfindungsgemäßen Verbindungen sind zur Herstellung pharmazeutischer Zubereitungen geeignet . Die pharmazeutischen Zubereitungen können eine oder mehrere der erfindungsgemäßen Verbindungen enthalten . Zur Herstellung der pharmazeutischen Zubereitungen beziehungsweise therapeutisch anwendbaren Formen können die üblichen physiologisch verträglichen Verdünnungsmittel, Träger- und Hilfsstoffe verwendet werden.

Erfindungsgemäß werden Verbindungen der Formel I hergestellt, indem man Verbindungen der Formel

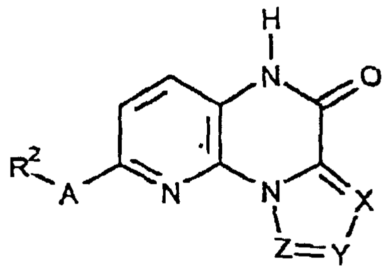

II

worin X, Y, Z, A und $R^2$ die angeführte Bedeutung haben , mit $R^1$ - Hal (Hal = Halogen) in Gegenwart eines anorganischen oder organischen basischen Katalysators umsetzt, wobei $R^1$ die ausgeführte Bedeutung besitzt

Das Verfahren kann ohne Lösungsmittel oder in einem geeigneten Lösungs- oder Dispergiermittel durchgeführt werden. Als Lösungs- oder Dispergiermittel kommen zum Beispiel in Betracht : Aromatische Kohlenwasserstoffe wie Benzol Toluol, Xylol, Mesitylen ; niedere aliphatische Ketone wie Aceton, Methylethylketon , Diethylketon; Ether wie Diethylether, Tetrahydrofuran, Dioxan Sulfoxide wie Dimethylsulfoxid ; tertiäre Säureamide wie Dimethylformamid, Dimethylacetamid, Tetramethylhamstoff, Hexamethylphosphorsäuretriamid, N-Methylpyrrolidon ; halogenierte Kohlenwasserstoffe wie Chlorbenzol, Dichlorbenzol Tetrachlorkohlenstoff; niedere Alkohole wie Methanol , Ethanol, Isopropanol sowie Mischungen der genannten Mittel, gegebenenfalls auch mit Wasser.

Die Reaktion wird beispielsweise bei Temperaturen zwischen 20 und 200 °C , vorzugsweise 50 bis 130 °C durchgeführt.

Bei der Ausgangskomponente $R^1$ - Hal bedeutet Hal vorzugsweise Chlor, Brom oder Jod.

Die Umsetzung wird vorteilhaft in Gegenwart von säurebindenden Mitteln wie Alkalicarbonaten (Natriumcarbonat, Kaliumcarbonat), Alkaliacetaten. Alkalihyroxyden oder tertiären Basen ( Triethylamin , Pyridin) durchgeführt . Vorzugsweise werden die Ausgangskomponenten der Formel II in Form ihrer Metallsalze eingesetzt. Insbesondere kommen die Alkalisalze in Frage . Die Herstellung der Alkalisalze erfolgt beispielsweise durch Umsetzung mit den entsprechenden Alkalihydriden, Alkaliamiden, Alkalialkoholaten oder auch Alkalimetallen in einem Lösungsmittel (niederer Alkohol, aromatischer Kohlenwasserstoff, tertiäre Säureamide) oder mit wäßrigem Alkali (zum Beispiel NaOH).

Weiterhin werden erfindungsgemäß Verbindungen der Formel I hergestellt, indem man Verbindungen der Formel

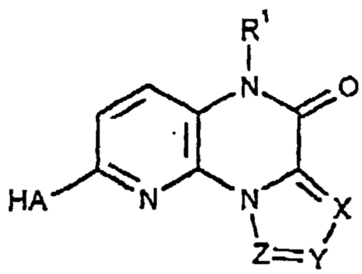

III

worin A, X, Y, Z und $R^1$ die angeführte Bedeutung haben, mit $R^2$ - Hal (Hal = Halogen) in Gegenwart eines anorganischen oder organischen basischen Katalysators umsetzt, wobei $R^2$ die ausgeführte Bedeutung besitzt.

Das Verfahren kann ohne Lösungsmittel oder in einem geeigneten Lösungs- oder Dispergiermittel durchgeführt werden. Als Lösungs- oder Dispergiermittel kommen zum Beispiel in Betracht. Aromatische Kohlenwasserstoffe

wie Benzol, Toluol, Xylol, Mesitylen ; niedere aliphatische Ketone wie Aceton , Methylethylketon, Diethylketon; Ether wie Diethylether Tetrahydrofuran, Dioxan; Sulfoxide wie Dimethylsulfoxid ; tertiäre Säureamide wie Dimethylformamid, Dimethylacetamid, Tetramethylhamstoff, Hexamethylphosphorsäuretriamid. N-Methylpyrrolidon ; halogenierte Kohlenwasserstoffe wie Chlorbenzol, Dichlorbenzol. Tetrachlorkohlenstoff; niedere Alkohole wie Methanol, Ethanol Isopropanol sowie Mischungen der genannten Mittel, gegebenenfalls auch mit Wasser.

Die Reaktion wird beispielsweise bei Temperaturen zwischen 20 und 200 °C, vorzugsweise bei 50 bis 130 °C durchgeführt.

Bei der Ausgangskomponente $R^2$ - Hal bedeutet Hal vorzugsweise Chlor, Brom oder Jod.

Die Umsetzung wird vorteilhaft in Gegenwart von säurebindenden Mitteln wie Alkalicarbonaten ( Natriumcarbonat, Kaliumcarbonat), Alkaliacetaten, Alkalihyroxyden oder tertiären Basen (Triethylamin, Pyridin) durchgeführt . Vorzugsweise werden die Ausgangskomponenten der Formel III in Form ihrer Metallsalze eingesetzt Insbesondere kommen die Alkalisalze in Frage. Die Herstellung der Alkalisalze erfolgt beispielsweise durch Umsetzung mit den entsprechenden Alkalihydriden Alkaliamiden, Alkalialkoholaten oder auch Alkalimetallen in einem Lösungsmittel (niederer Alkohol, aromatischer Kohlenwasserstoff, tertiäre Säureamide) oder mit wäßrigem Alkali (zum Beispiel NaOH).

Die neuen Pyrido[3,2-e]pyrazinone lassen sich mit anorganischen oder organischen Säuren oder Basen in Wasser oder in organischen Lösungsmitteln in die entsprechenden Salze überführen.

Die erfindungsgemäßen Verbindungen der Formel I und ihre Salze sind biologisch aktiv.

Die erfindungsgemäßen Verbindungen zeigen in-vitro eine starke Inhibition der Phosphodiesterase-Isoenzyme IV und V, eine starke Beeinflussung der durch Histamin präkontrahierten Trachea (Meerschweinchen) sowie gute Wirkungen in in-vivo Asthma-Modellen, wie zum Beispiel bei der asthmatischen Spätphase-Reaktion (Eosinophilie) im Meerschweinchen.

<u>Methoden:</u>

Bestimmung der Phosphodiesterase - Aktivität

Die Phosphodiesterase(PDE)-Aktivität wird mit einigen Modifizierungen ( Bauer, A.C.; Schwabe, U., An improved assay of cyclic 3',5'-nucleotide phosphodiesterase with QAE Sephadex A-25. Naunyn-Schmiedeberg's Arch. Pharmacol. <u>311</u> 193-198 (1980)) nach der von Thompson et al. beschriebenen Methode bestimmt. (Thompson, W.J. ; Appleman, M.M., Assay of cyclic nucleotide phosphodiesterase and resolution of multiple molecular forms of the enzyme. Adv. Cycl. Nucl. Res. <u>10</u> 69-92 (1979)) Die Reaktionsmischung enthält 40 mM Tris-HCl (pH 7,4), 5 mM $MgCl_2$, 0,5 µM cAMP oder cGMP, [$^3$H] cAMP oder [$^3$H] cGMP (ca. 20.000 cpm/Test) und die zur Erfassung der einzelnen Isoenzyme notwendigen weiteren Komponenten (siehe unten). Das Endvolumen beträgt 200 µl. Testsubstanzen werden als Stammlösungen in DMSO angesetzt Die DMSO-Konzentration im Reaktionsgemisch ist gleich oder weniger als 1% v/v. Bei dieser DMSO-Konzentration wird die PDE-Aktivität nicht beeinflußt Nach einer 5-minütigen Vorinkubation bei 37 °C wird die Reaktion durch Zugabe des Substrates (cAMP oder cGMP) gestartet Die Proben werden für weitere 15 Minuten bei 37°C inkubiert. Durch Zugabe von 50 µl 0,2 N HCl wird die Reaktion gestoppt. Die Proben bleiben für weitere 10 Minuten im Eis. Nach der Inkubation mit 25 µg 5'-Nukleotidase (Crotalus atrox) für 10 Minuten bei 37°C werden die Proben auf QAE Sephadex A-25-Säulen (Econor columns, Bio-Rad) aufgetragen. Die Säulen werden mit 2 ml 30 mM Ammoniumformiat (pH 6,0) eluiert. Die Radioaktivität der einzelnen Fraktionen wird scintigraphisch erfaßt.

Die PDE IV ( cAMP-spezifisch) - Aktivität wird nach der von Schudt et al. (Schudt C. ; Winter S.; Forderkurz S.; Hakelmann A.; Ullrich V. Influence of selective phosphodiesterase inhibitors on human neutrophil functions and levels of cAMP and Ca . Naunyn-Schmiedeberg's Arch. Pharmacol. <u>344</u> ,682-690 (1991)) beschriebenen Methode im Cytosol menschlicher polymorphkerniger Leukozyten bestimmt. Substrat ist cAMP. Durch Zugabe von Motapizon einem spezifischen PDE III - Inhibitor (1 µM) wird die aus der möglichen thrombozytischen Verunreinigung stammende PDE III - Aktivität vollkommen unterdrückt.

PDE V (cGMP spezifisch) wird aus Human-Blutplättchen isoliert ( Schudt C.; Winder S.; Müller B.; Ukena D., Zardaverine as a selective inhibitor of phosphodiesterase isoenzymes. Biochem. Pharmacol. <u>42</u>, 153-162 (1991)). Als Substrat wird cGMP eingesetzt

Beeinflussung der durch Histamin präkontrahierten Trachea

Meerschweinchen werden in Narkose entblutet . Anschließend wird die Trachea vom anliegenden Gewebe freipräpariert und in fünf gleiche Teile (mindestens 3 Tracheaspangen breit ) geschnitten. Die Tracheateile werden in ein Bad, das mit einer Nährlösung (Krebs-Henseleit) gefüllt ist, eingehängt. Mit Hilfe von Kraftaufnehmem kann die Stärke der Kontraktion der Trachea gemessen werden. Nach dem Einhängen wird 15 Minuten zur Eingewöhnung

gewartet. Danach wird mit Isoprenalin (1x $10^{-7}$mol/l) die Trachea vollständig entspannt. Anschließend wird das Badgefäß gespült. Mit Metacholin (10x$10^{-5}$ mol/l) wird ein Kontraktionsmaximum ausgelöst. Danach wird das Badgefäß erneut gespült. Es wird dann Histamin (1x$10^{4}$ mol/l zugegeben . Nach ca. 10 Minuten wird das Kontraktionsmaximum erreicht . Jetzt wird in steigender Konzentration die Prüfsubstanz ins Bad gegeben und die kontraktionslösende Wirkung in Prozent zu einer unbehandelten Kontrolle ermitteln. Die mittlere kontraktionslösende Konzentration wird mit Regressionsgeräten berechnet . Zur Funktionskontrolle der Organe wird am Ende des Versuches nochmals Isoprenalin (1x$10^{-5}$ mol/l) in das Bad gegeben , um zu sehen, ob sich die Organe noch entspannen können.

Bestimmung der asthmatischen Spätphase-Reaktion (Eosinophilie) in Meerschweinchen

Männliche Meerschweinchen (250 - 300 g, Pirbright white, Charles River Wiga) werden durch s.c. Injektion von Ovalburnin (10 µg + 100 mg Aluminiumhydroxid) aktiv sensibilisiert und 2 Wochen später geboostert. Eine Woche nach der Boosterung (10 µg + 100 mg Aluminiumhydroxid) werden die Tiere einem Aerosol aus vemebelter 0,5 %iger Ovalbumin - Lösung für 20 Sekunden ausgesetzt. 24 Stunden später wird an den durch eine Überdosis Pentobarbital getöteten Tieren die bronchoalveoläre Lavage (BAL) mit 2 x 5 ml Kochsalzlösung durchgeführt. Die Lavage - Flüssigkeit wird gepoolt und 10 Minuten zentrifugiert. Das Zellpellet wird in 1 ml physiologischer Kochsalzlösung suspendiert und die Eosinophilen mittels Becton-Dickinson Eosinophilen Kit mikroskopisch in einer Zählkammer ausgezählt. Für jedes Meerschweinchen werden die Eosinophilen gezählt. Für jede Gruppe wird der Mittelwert berechnet. Die Hemmung der Eosinophilie für die substanzbehandelte Gruppe ergibt sich nach der Formel

$$(A - C) - (B - C)/ (A - C) \times 100 = \% \text{ Hemmung}$$

A = Eosinophilie in der nicht behandelten, mit Ovalbumin gechallengten Kontroll-Gruppe
B = Eosinophilie in der mit der Substanz behandelten und mit Ovalbumin gechallengten Gruppe
C = Eosinophilie in der nicht mit Ovalbumin gechallengten Kontrollgruppe

Die Substanzapplikation erfolgt 2 Stunden vor Allergen - Challenge p.o. (in 1% Methocel) oder i.p. (in 0,5% Methocel). Die Kontrollgruppen erhalten 1% Methocel p.o. oder 0,5% Methocel i.p. 2 Stunden vor Allergen - Challenge .
Beispielsweise werden für die Verbindung gemäß Ausführungsbeispiel 1 folgende Wirkungen ermittelt :

PDE (V- Inhibition (in-vitro): $IC_{50}$ = 0,1 µmol/l
PDE V - Inhibition (in-vitro): $IC_{50}$ = 0,095 µmol/l
Histamin präkontrahierte Trachea : $IC_{50}$ = 0,7 µmol/l
Ovalbumin-induzierte Eosinophilie (Meerschweinchen) : 1 mg/kg i.p. 74 % Hemmung .

**Ausführungsbeispiele**

Beispiele zur Herstellung von Verbindungen der Formel I aus Verbindungen der Formel II :

Beispiel 1: 1-Ethyl-8-methoxy-3-methyl-5-propyl-imidazo[1,5-a]pyrido[3,2-e]pyrazinon

Variante A:

10 g (0,038 mol) 1-Ethyl-8-methoxy-3-methyl-imidazo[1,5-a]pyrido[3,2-e]pyrazinon werden in 200 ml Dimethylformamid eingerührt. Bei 20 °C werden unter Rühren 3 g (0,095 mol) Natriumhydrid (80 %ig) portionsweise zugegeben . Nachdem das Gemisch 2 Stunden gerührt wurde, werden 8,5 g (0,07 mol) n-Propylbromid innerhalb von 15 Minuten zugetropft. Die entstehende Lösung wird unter Rühren 2 Stunden auf 70 - 80 °C erwärmt und anschließend weitere 8 Stunden auf 100 °C erhitzt. Nach Abkühlung auf 20 °C wird das Lösungsmittel im Vakuum entfernt. Das dabei kristallisierende Rohprodukt wird zunächst mit 150 ml ca. 50 °C warmem Wasser ausgerührt und anschließend aus Cyclohexan umkristallisiert.
Ausbeute : 8,5 g (73 % d. Th.)
Schmelzpunkt: 136 - 137°C

Variante B :

10 g (0,038 mol) 1-Ethyl-8-methoxy-3-methyl-imidazo[1,5-a]pyrido[3,2-e]pyrazinon werden in 200 ml Dimethylacetamid eingerührt. Bei 20 °C werden unter Rühren 3 g (0,095 mol) Natriumhydrid (80 %ig) portionsweise zugegeben. Nachdem das Gemisch 2 Stunden gerührt wurde, werden 8,5 g (0,07 mol) n-Propylbromid innerhalb von 15 Minuten zugetropft. Die entstehende Lösung wird noch 15 Stunden bei 20-25 °C gerührt. Anschließend wird das Lösungsmittel im Vakuum entfernt. Die Reinigung des kristallisierenden Rohproduktes erfolgt wie bei Variante A beschrieben.
Ausbeute : 8,1 g (70 % d. Th.)
Schmelzpunkt : 135 - 137 °C

Variante C

10 g (0,038 mol) 1-Ethyl-8-methoxy-3-methyl-imidazo[1,5-a]pyrido[3,2-e]pyrazinon werden mit 6,9 g (0,05 mol) wasserfreiem Kaliumkarbonat in 80 ml Dimethylformamid unter Rühren 1 Stunde auf 120 °C erhitzt. Danach werden unter Rühren 8,5 g (0,07 mol) n-Propylbromid innerhalb von 15 Minuten zugetropft. Das Reaktionsgemisch wird 7 Stunden bei 120 -130 °C gerührt. Nach dem Erkalten werden die anorganischen Salze abgesaugt und vom Filtrat das Lösungsmittel im Vakuum entfernt. Zur Reinigung des kristallisierenden Rohproduktes wird aus Cyclohexan umkristallisiert.
Ausbeute : 8,0 g (69 % d. Th.)
Schmelzpunkt : 135 - 137 °C
Unter Verwendung der angegebenen beispielhaften Varianten können zahlreiche weitere Verbindungen der Formel I hergestellt werden, von denen folgende beispielhaft angeführt werden:

| Beispiel | X | Y | Z | A | $R^1$ | $R^2$ | Variante | Ausbeute [ % ] | Schmelzpkt. [°C] |
|---|---|---|---|---|---|---|---|---|---|
| 2 | $C-CH_3$ | N | $C-C_2H_5$ | O | $CH_3$ | H | B | 92 | 276 - 278 Ethanol |
| 3 | $C-CH_3$ | N | $C-C_2H_5$ | O | $C_2H_5$ | H | B | 90 | 157 - 160 Ethanol |
| 4 | $C-CH_3$ | N | $C-C_2H_5$ | O | $C_3H_7$ | H | B | 77 | 295 Ethanol |
| 5 | $C-CH_3$ | N | $C-C_2H_5$ | O | $CH_3$ | $CH_3$ | B | 74 | 173 DMF |
| 6 | $C-CH_3$ | N | C-H | O | $CH_3$ | $CH_3$ | B | 76 | 254 Essigester |
| 7 | C-H | N | $C-CH_3$ | O | $CH_3$ | $CH_3$ | B | 80 | 279 DMF |
| 8 | $C-CH_3$ | N | $C-C_2H_5$ | O | $C_2H_5$ | $CH_3$ | B | 68 | 145 - 147 DMF |
| 9 | $C-CH_3$ | N | C-H | O | $C_2H_5$ | $CH_3$ | B | 67 | 177 Essigester |
| 10 | $C-CH_3$ | N | $C-C_2H_5$ | O | $C_4H_9$ | $CH_3$ | C | 54 | 99 - 102 Cyclohexan |
| 11 | $C-CH_3$ | N | $C-C_2H_5$ | O | $C_5H_{11}$ | $CH_3$ | A | 33 | 72 - 74 Cyclohexan |
| 12 | $C-CH_3$ | N | $C-C_2H_5$ | O | $(CH_2)_2CH(CH_3)_2$ | $CH_3$ | A | 11 | 113 - 116 Cyclohexan |
| 13 | $C-CH_3$ | N | $C-C_2H_5$ | O | $CH_2C_6H_5$ | $CH_3$ | B | 44 | 166 - 167 Aceton |
| 14 | $C-CH_3$ | N | $C-C_2H_5$ | O | $C_2H_4C_6H_5$ | $CH_3$ | C | 10 | 174 - 176 Aceton |
| 15 | $C-CH_3$ | N | $C-C_2H_5$ | O | $CH_2C_6H_4(2-Cl)$ | $CH_3$ | A | 58 | 245 - 246 DMF |

| Beispiel | X | Y | Z | A | R1 | R2 | Variante | Ausbeute [ % ] | Schmelzpkt. [°C] |
|---|---|---|---|---|---|---|---|---|---|
| 16 | $C-CH_3$ | N | $C-C_2H_5$ | O | $CH_2C_6H_4$(4-Cl) | $CH_3$ | A | 64 | 201 - 202 DMF |
| 17 | $C-CH_3$ | N | $C-C_2H_5$ | O | $CH_2C_6H_3$(2,4-di-Cl) | $CH_3$ | C | 17 | 211 - 213 Aceton |
| 18 | $C-CH_3$ | N | $C-C_2H_5$ | O | $CH_2C_6H_3$(2,6-di-Cl) | $CH_3$ | A | 33 | 209 - 212 Toluol |
| 19 | $C-CH_3$ | N | $C-C_2H_5$ | O | $CH_2C_6H_4$(2-F) | $CH_3$ | A | 51 | 186 - 187 Ethanol |
| 20 | $C-CH_3$ | N | $C-C_2H_5$ | O | $CH_2C_6H_4$(4-F) | $CH_3$ | A | 60 | 189 - 191 DMF |
| 21 | $C-CH_3$ | N | $C-C_2H_5$ | O | $CH_2C_6H_3$(2-Cl,6-F) | $CH_3$ | A | 26 | 197 - 200 Aceton |
| 22 | $C-CH_3$ | N | $C-C_2H_5$ | O | $CH_2C_6H_4(2-CH_3)$ | $CH_3$ | A | 50 | 240 - 242 Toluol |
| 23 | $C-CH_3$ | N | $C-C_2H_5$ | O | $CH_2C_6H_4(4-OCH_3)$ | $CH_3$ | A | 61 | 156 - 158 Ethanol |
| 24 | $C-CH_3$ | N | $C-C_2H_5$ | O | $CH_2C_6H_2(3,4,5-tri-OCH_3)$ | $CH_3$ | A | 69 | 191 - 192 Ethanol |
| 25 | $C-CH_3$ | N | $C-C_2H_5$ | O | $CH_2C_6H_4(4-OCH_2-C_6H_5)$ | $CH_3$ | A | 54 | 147 - 149 Ethanol |
| 26 | $C-CH_3$ | N | $C-C_2H_5$ | O | $(CH_2)_3COOC_2H_5$ | $CH_3$ | B | 57 | 130 - 132 Isopropanol |
| 27 | $C-CH_3$ | N | $C-C_2H_5$ | O | $(CH_2)_3COONa$ | $CH_3$ | B | 65 | 293 - 295 Ethanol |
| 28 | $C-CH_3$ | N | $C-C_2H_5$ | O | $C_3H_7$ | $C_2H_5$ | B | 67 | 124 - 126 Ethanol |
| 29 | $C-CH_3$ | N | $C-C_2H_5$ | O | $C_2H_5$ | $CH_2COCH_3$ | B | 70 | 174 - 176 Ethanol |

Beispiele zur Herstellung von Verbindungen der Formel I aus Verbindungen der Formel III :

Beispiel 30 : 8-Cyclopentyloxy-1-ethyl-methyl-5-propyl-imidazo[1,5-a]pyrido[3,2-e]pyrazinon

Variante A :

3,2 g (0,011 mol) 1-Ethyl-8-hydroxy-3-methyl-imidazo[1,5-a]pyrido[3,2-e]pyrazinon werden in 60 ml Dimethylformamid eingerührt. Bei 20 °C werden unter Rühren 0,9 g (0,03 mol) Natriumhydrid (80 %ig) portionsweise zugegeben . Nachdem das

Gemisch 2 Stunden gerührt wurde, werden 2,1 g (0,02 mol) Cyclopentylchlorid innerhalb von 15 Minuten zugetropft. Die entstehende Lösung wird unter Rühren 2 Stunden auf 70 - 80 °C erwärmt und anschließend weitere 8 Stunden auf 100 °C erhitzt . Nach Abkühlung auf 20 °C wird das Lösungsmittel im Vakuum entfernt. Das dabei kristallisierende Rohprodukt wird zunächst mit 50 ml ca. 50 °C warmem Wasser ausgerührt und anschließend aus Essigester umkristallisiert .
Ausbeute : 3,0 g (77 % d. Th.)
Schmelzpunkt : 138 - 140 °C

Variante B:

3,2 g (0,011 mol) 1-Ethyl-8-hydroxy-3-methyl-imidazo[1,5-a]pyrido[3,2-e]pyrazinon werden in 60 ml Dimethylacetamid eingerührt. Bei 20 °C werden unter Rühren 0,9 g (0,03 mol) Natriumhydrid (80 %ig) portionsweise zugegeben . Nachdem das Gemisch 2 Stunden gerührt wurde, werden 2,1 g (0,02 mol) Cyclopentylchlorid innerhalb von 15 Minuten zugetropft. Die entstehende Lösung wird noch 15 Stunden bei 20 - 25 °C gerührt . Anschließend wird das Lösungsmittel im Vakuum entfernt. Die Reinigung des kristaltisierenden Rohproduktes erfolgt wie bei Variante A beschrieben.
Ausbeute: 2.7 g (70 % d. Th.)
Schmelzpunkt: 138-140°C

Unter Verwendung der angegebenen beispielhaften Varianten können zahlreiche weitere Verbindungen der Formel I hergestellt werden, von denen folgende beispielhaft angeführt werden;

| Beispiel | X | Y | Z | A | $R^1$ | $R^2$ | Variante | Ausbeute [%] | Schmelzpkt. [°C] |
|---|---|---|---|---|---|---|---|---|---|
| 31 | $C-CH_3$ | N | $C-C_2H_5$ | O | $CH_3$ | $C_2H_5$ | B | 97 | 216 DMF |
| 32 | $C-CH_3$ | N | $C-C_2H_5$ | O | $C_2H_5$ | $C_2H_5$ | A | 43 | 132 - 134 Isopropanol |
| 33 | $C-CH_3$ | N | $C-C_2H_5$ | O | $CH_3$ | $CH_2COCH_3$ | B | 61 | 174 - 175 Ethanol |
| 34 | $C-CH_3$ | N | $C-C_2H_5$ | O | $CH_3$ | $(CH_2)_3COCH_3$ | B | 22 | 142 - 143 Ethanol |
| 35 | $C-CH_3$ | N | $C-C_2H_5$ | O | $C_2H_5$ | $(CH_2)_2CH_2OH$ | B | 24 | 140 - 142 Isopropanol |
| 36 | $C-CH_3$ | N | $C-C_2H_5$ | O | $C_2H_5$ | $(CH_2)_2CH_2SO_3H$ | B | 67 | 336 - 337 Isopropanol |
| 37 | $C-CH_3$ | N | $C-C_2H_5$ | O | $CH_3$ | $(CH_2)_3COOH$ | B | 37 | 233 - 235 Isopropanol |
| 38 | $C-CH_3$ | N | $C-C_2H_5$ | O | $C_2H_5$ | $(CH_2)_3COOH$ | B | 70 | 165 Ethanol |
| 39 | $C-CH_3$ | N | $C-C_2H_5$ | O | $CH_3$ | $(CH_2)_3COOC_2H_5$ | B | 94 | 140 - 141 DMF |
| 40 | $C-CH_3$ | N | $C-C_2H_5$ | O | $C_2H_5$ | $(CH_2)_3COOC_2H_5$ | B | 17 | 78 Ethanol |
| 41 | $C-CH_3$ | N | $C-C_2H_5$ | O | $C_2H_5$ | $(CH_2)_3CON(CH_3)C_6H_{11}$ | B | 11 | 133 - 135 Essigester |
| 42 | $C-CH_3$ | N | $C-C_2H_5$ | O | $CH_3$ | $CH_2C_6H_5$ | B | 70 | 165 DMF |
| 43 | $C-CH_3$ | N | $C-C_2H_5$ | O | $C_2H_5$ | $CH_2C_6H_5$ | B | 27 | 147 - 148 Isopropanol |
| 44 | $C-CH_3$ | N | $C-C_2H_5$ | O | $CH_3$ | $-CH_2C_6H_4(4\text{-}F)$ | B | 67 | 223 Isopropanol |

| Beispiel | X | Y | Z | A | R1 | R2 | Variante | Ausbeute [%] | Schmelzpkl. [°C] |
|---|---|---|---|---|---|---|---|---|---|
| 45 | C-$CH_3$ | N | C-$C_2H_5$ | O | $C_2H_5$ | $CH_2C_6H_4$(4-F) | B | 52 | 148 - 150 Isopropanol |
| 46 | C-$CH_3$ | N | C-$C_2H_5$ | O | $CH_2C_6H_2$(3,4,5-tri-$OCH_3$) | $CH_2C_6H_2$(3,4,5-tri-$OCH_3$) | A | 52 | 172 - 174 Cyclohexan |
| Vergleich 47 | C-$CH_3$ | N | C-$C_2H_5$ | NH | H | $CH_2C_6H_5$ | B | 40 | 276 - 278 Ethanol |
| 48 | C-$CH_3$ | N | C-$C_2H_5$ | O | $CH_3$ | 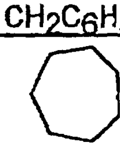 | B | 9 | 173 - 175 Isopropanol |
| 49 | C-$CH_3$ | N | C-$C_2H_5$ | O | $CH_3$ | 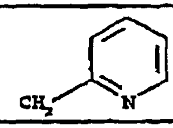 | B | 3 | 184 - 186 Ethanol |
| 50 | C-$CH_3$ | N | C-$C_2H_5$ | O | $CH_3$ | 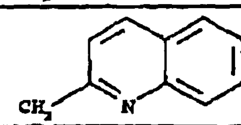 | B | 18 | 237 - 239 DMF |
| 51 | C-$CH_3$ | N | C-$C_2H_5$ | O | $C_2H_5$ | 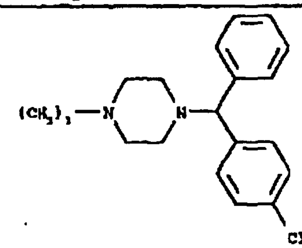 | A | 13 | 54 - 56 Cyclohexan |

**Patentansprüche**

**1.** Pyrido[3,2-e]pyrazinone der Formel

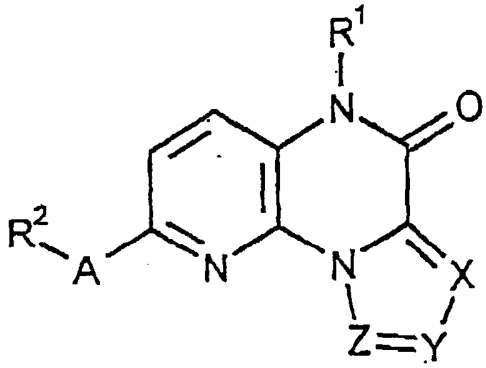

I

worin

A Sauerstoff bedeutet;
X, Y und Z für N oder $CR^3$ stehen, wobei mindestens einer von X, Y und Z N bedeuten muss;

$R^1$ $C_1$-$C_{10}$-Alkyl (gegebenenfalls auch verzweigt), das ein- oder mehrfach substituiert sein kann mit Hydroxy-, $C_1$-$C_6$-Alkyloxy-, Alkenyloxy- mit bis 6 C-Atomen, Alkinyloxy- mit bis 6 C-Atomen, Aryl-, Aryloxy-, Heteroaryl-, Heteroaryloxy-, Amino-, Halogen, $NO_2$, CN, C=$OR^4$ oder $S(O)_nR^5$ (mit n von 0 bis 2); Alkenyl mit bis 10 C-Atomen (gegebenenfalls auch verzweigt), das ein- oder mehrfach substituiert sein kann mit Hydroxy-, $C_1$-$C_6$-Alkyloxy-, Alkenyloxy- mit bis 6 C-Atomen, Alkinyloxy- mit bis 6 C-Atomen, Aryl-, Aryloxy-, Heteroaryl-, Heteroaryloxy-, Amino, Halogen, $NO_2$, CN, C = $OR^4$ oder $S(O)_nR^5$ (mit n von 0 bis 2); oder
$C_5$-$C_7$-Cycloalkyl, das ein- oder mehrfach substituiert sein kann mit Hydroxy-, $C_1$-$C_6$-Alkyloxy-, Alkenyloxy- mit bis zu 6 C-Atomen, Alkinyloxy- mit bis 6 C-Atomen, Aryl-, Aryloxy-, Heteroaryl- Heteroaryloxy-, Amino-, Halogen, $NO_2$, CN, C = $OR^4$ oder $S(O)_nR^5$ (mit n von 0 bis 2) darstellt;

$R^2$ für H; $C_1$-$C_{10}$-Alkyl (gegebenenfalls auch verzweigt), das ein- oder mehrfach substituiert sein kann mit Hydroxy-, $C_1$-$C_6$-Alkyloxy, Alkenyloxy-mit bis 6 C-Atomen, Alkinyloxy- mit bis 6 C-Atomen, Aryl-, Aryloxy-, Heteroaryl-, Heteroaryloxy-, Amino-, Halogen, $NO_2$, CN, C=$OR^4$ oder $S(O)_nR^5$ (mit n von 0 bis 3); Alkenyl mit bis 10 C-Atomen (gegebenenfalls auch verzweigt), das ein- oder mehrfach substituiert sein kann mit Hydroxy-, $C_1$-$C_6$-Alkyloxy-, Alkenyloxy- mit bis 6 C-Atomen, Alkinyloxy- mit bis 6 C-Atomen, Aryl-, Aryloxy-, Heteroaryl-, Heteroaryloxy-, Amino-, Halogen, $NO_2$, CN, C = $OR^4$ oder $S(O)_nR^5$ (mit n von 0 bis 2); oder
$C_5$-$C_7$-Cycloalkyl, das ein- oder mehrfach substituiert sein kann mit Hydroxy-, $C_1$-$C_6$-Alkyloxy-, Alkenyloxy- mit bis 6 C-Atomen, Alkinyloxy- mit bis 6 C-Atomen, Aryl-, Aryloxy-, Heteroaryl-, Heteroaryloxy-, Amino-, Halogen, $NO_2$, CN, C=$OR^4$ oder $S(O)_nR^5$ (mit n von O bis 2) stehen kann;

$R^3$ H; $C_1$-$C_6$-Alkyl (gegebenenfalls verzweigt) bedeutet;

$R^4$ für H; $C_1$-$C_6$-Alkyl (gegebenenfalls verzweigt); Phenyl; OH; $C_1$-$C_6$-Alkyloxy (gegebenenfalls verzweigt); Aryloxy; Amino steht;

$R^5$ H; $C_1$-$C_6$-Alkyl; Aryl; OH; $C_1$-$C_6$-Alkyloxy; Aryloxy; Amino bedeutet und ihre physiologisch verträglichen Salze sowie Verbindungen worin

| X | Y | Z | A | $R^1$ | $R^2$ |
|---|---|---|---|---|---|
| $C\text{-}CH_3$ | N | $C\text{-}C_2H_5$ | O | $CH_2C_6H_4$(2-Cl) | $CH_3$ |
| $C\text{-}CH_3$ | N | $C\text{-}C_2H_5$ | O | $CH_2C_6H_4$(4-Cl) | $CH_3$ |
| $C\text{-}CH_3$ | N | $C\text{-}C_2H_5$ | O | $CH_2C_6H_3$(2,4-di-Cl) | $CH_3$ |
| $C\text{-}CH_3$ | N | $C\text{-}C_2H_5$ | O | $CH_2C_6H_3$(2,6-di-Cl) | $CH_3$ |
| $C\text{-}CH_3$ | N | $C\text{-}C_2H_5$ | O | $CH_2C_6H_4$(2-F) | $CH_3$ |
| $C\text{-}CH_3$ | N | $C\text{-}C_2H_5$ | O | $CH_2C_6H_4$(4-F) | $CH_3$ |
| $C\text{-}CH_3$ | N | $C\text{-}C_2H_5$ | O | $CH_2C_6H_3$(2-Cl,6-F) | $CH_3$ |
| $C\text{-}CH_3$ | N | $C\text{-}C_2H_5$ | O | $CH_2C_6H_4$(2-$CH_3$) | $CH_3$ |
| $C\text{-}CH_3$ | N | $C\text{-}C_2H_5$ | O | $CH_2C_6H_4$(4-$OCH_3$) | $CH_3$ |
| $C\text{-}CH_3$ | N | $C\text{-}C_2H_5$ | O | $CH_2C_6H_2$(3,4,5-tri-$OCH_3$) | $CH_3$ |
| $C\text{-}CH_3$ | N | $C\text{-}C_2H_5$ | O | $CH_2C_6H_4$(4-$OCH_2$-$C_6H_5$) | $CH_3$ |
| $C\text{-}CH_3$ | N | $C\text{-}C_2H_5$ | O | $C_2H_5$ | $(CH_2)_3CON(CH_3)C_6H_{11}$ |
| $C\text{-}CH_3$ | N | $C\text{-}C_2H_5$ | O | $CH_3$ | $CH_2C_6H_4$(4-F) |

| X | Y | Z | A | $R^1$ | $R^2$ |
|---|---|---|---|---|---|
| $C\text{-}CH_3$ | N | $C\text{-}C_2H_5$ | O | $C_2H_5$ | $CH_2C_6H_4(4\text{-}F)$ |
| $C\text{-}CH_3$ | N | $C\text{-}C_2H_5$ | O | $CH_2C_6H_2(3,4,5\text{-tri-}OCH_3)$ | $CH_2C_6H_2(3,4,5\text{-tri-}OCH_3)$ |
| $C\text{-}CH_3$ | N | $C\text{-}C_2H_5$ | O | $C_2H_5$ | 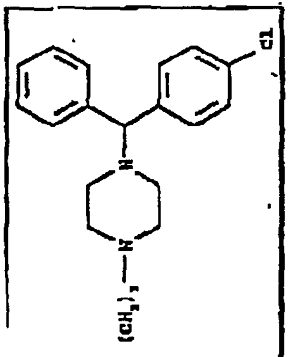 |

2. Verfahren zur Herstellung Pyrido[3,2-e]pyrazinone der Formel I, worin A, X, Y, Z, $R^1$, $R^2$, $R^3$, $R^4$ und $R^5$ die im Anspruch 1 genannte Bedeutung haben, **gekennzeichnet dadurch, daß** Verbindungen der Formel

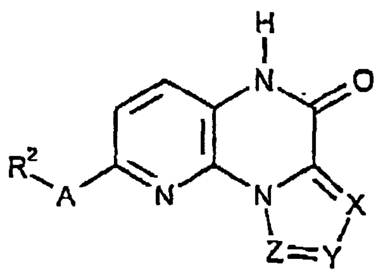

II

worin X, Y, Z, A und $R^2$ die im Anspruch 1 angeführte Bedeutung haben , mit $R^1$ - Hal (Hal = Halogen) in Gegenwart eines anorganischen oder organischen basischen Katalysators umgesetzt werden , wobei $R^1$ die im Anspruch 1 ausgeführte Bedeutung besitzt.

3. Verfahren zur Herstellung Pyrido[3,2-e]pyrazinone der Formel I, worin A, X, Y , Z, $R^1$, $R^2$, $R^3$, $R^4$ und $R^5$ die im Anspruch 1 genannte Bedeutung haben , **gekennzeichnet dadurch , daß** Verbindungen der Formel

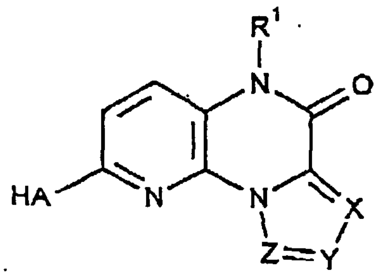

III

worin A, X , Y, Z und $R^1$ die im Anspruch 1 angeführte Bedeutung haben , mit $R^2$ - Hal (Hal = Halogen) in Gegenwart eines anorganischen oder organischen basischen Katalysators umgesetzt werden., wobei $R^2$ die im Anspruch 1 ausgeführte Bedeutung besitzt.

4. Verfahren nach einem oder mehreren vorangegangenen Ansprüchen, **dadurch gekennzeichnet, daß** man basische Verbindungen der Formel I in Salze überführt.

5. Verfahren nach einem oder mehreren vorangegangenen Ansprüchen, **dadurch gekennzeichnet, daß** man saure Verbindungen der Formel I in Salze überführt.

6. Verbindungen der Formel I nach Anspruch 1 zur Anwendung als therapeutische Wirkstoffe mit antiasthmatischen und antiallergischen Wirkungen.

7. Arzneimittel, enthaltend eine oder mehrere Verbindung der Formel I nach Anspruch 1 neben üblichen physiologisch verträglichen Trägern und/oder Verdünnungsmitteln beziehungsweise Hilfsstoffen.

8. Verfahren zur Herstellung eines Arzneimittels nach Anspruch 7, **gekennzeichnet dadurch , daß** eine oder mehrere Verbindung der Formel I mit gebräuchlichen pharmazeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht werden.

9. Verwendung von Verbindungen der allgemeinen Formel I nach Anspruch 1 zur Herstellung von Arzneimitteln nach den Ansprüchen 7 und 8 zur Anwendung bei asthmatischen und allergischen Erkrankungen allein oder in Kombination untereinander oder in Kombination mit Trägerstoffen und/oder Verdünnungsmitteln beziehungsweise sonstigen Hilfsstoffen.

## Claims

1. Pyrido [3,2-e] pyrazinones of formula

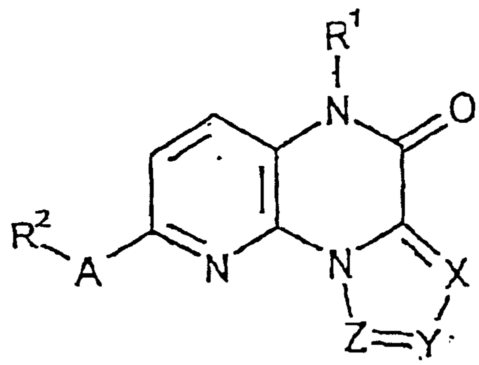

I

wherein

A represents oxygen;
X, Y and Z represent N or $CR^3$, wherein at least one of X, Y and Z must represent N;

$R^1$ represents $C_1$ to $C_{10}$ alkyl (optionally also branched), which may represent singly or multiply substituted with hydroxy, $C_1$ to $C_6$ alkyloxy, alkenyloxy with 6 carbon atoms, alkinyloxy with up to 6 carbon atoms, aryl, aryloxy, heteroaryl, heteroaryloxy, amino, halogen, $NO_2$, CN, $C=OR^4$ or $S(O)_nR^5$ (where n is from 0 to 2) ;
alkenyl with up to 10 carbon atoms (optionally also branched), which may represent singly or multiply substituted with hydroxy, $C_1$ to $C_6$ alkyloxy, alkenyloxy with 6 carbon -atoms, alkinyloxy with up to 6 carbon atoms, aryl, aryloxy, heteroaryl, heteroaryloxy, amino, halogen, $NO_2$, CN, $C=OR^4$ or $S(O)_nR^5$ (where n represents from 0 to 2) ; or
$C_5$ to $C_7$ cycloakyl, which may represent singly or multiply substituted with hydroxy, $C_1$ to $C_6$ alkyloxy, alkenyloxy with 6 carbon atoms, alkinyloxy with up to 6 carbon atoms, aryl, aryloxy, heteroaryl, heteroaryloxy, amino, halogen, $NO_2$, CN, $C=OR^4$ or $S(O)_nR^5$ (where n represents from 0 to 2);

$R^2$ may represent H; $C_1$ to $C_{10}$ alkyl (optionally also branched), which may represent singly or multiply substituted with hydroxy, $C_1$ to $C_6$ alkyloxy, alkenyloxy with 6 carbon atoms, alkinyloxy with up to 6 carbon atoms, aryl, aryloxy, heteroaryl, heteroaryloxy, amino, halogen, $NO_2$, CN, $C=OR^4$ or $S(O)_nR^5$ (where n represents from 0 to 3) ;
alkenyl with up to 10 carbon atoms (optionally also branched), which may represent singly or multiply substituted with hydroxy, $C_1$ to $C_6$ alkyloxy, alkenyloxy with 6 carbon atoms, alkinyloxy with up to 6 carbon atoms, aryl, aryloxy, heteroaryl, heteroaryloxy, amino, halogen, $NO_2$, CN, $C=OR^4$ or $S(O)_nR^5$ (where n represents from 0 to 2); or
$C_5$ to $C_7$ cycloalkyl which may represent singly or multiply substituted with hydroxy, $C_1$ to $C_6$ alkyloxy, alkenyloxy with 6 carbon atoms, alkinyloxy with up to 6 carbon atoms, aryl, aryloxy, heteroaryl, heteroaryloxy, amino, halogen, $NO_2$, CN, $C=OR^4$ or $S(O)_nR^5$ (where n represents from 0 to 2) ;

$R^3$ represents H; $C_1$ to $C_6$ alkyl (optionally branched) ;

$R^4$ represents H; $C_1$ to $C_6$ alkyl (optionally branched); phenyl; OH; $C_1$ to $C_6$ alkyloxy (optionally branched); aryloxy; amino;

$R^5$ represents H; $C_1$ to $C_6$ alkyl; aryl; OH; $C_1$ to $C_6$ alkyloxy; aryloxy; amino and their physiologically compatible salts and

compounds, wherein

| X | Y | Z | A | $R^1$ | $R^2$ |
|---|---|---|---|---|---|
| $C-CH_3$ | N | $C-C_2H_5$ | O | $CH_2C_6H_4(2-Cl)$ | $CH_3$ |
| $C-CH_3$ | N | $C-C_2H_5$ | O | $CH_2C_6H_4(4-Cl)$ | $CH_3$ |
| $C-CH_3$ | N | $C-C_2H_5$ | O | $CH_2C_6H_3(2,4-di-Cl)$ | $CH_3$ |
| $C-CH_3$ | N | $C-C_2H_5$ | O | $CH_2C_6H_3(2,6-di-Cl)$ | $CH_3$ |
| $C-CH_3$ | N | $C-C_2H_5$ | O | $CH_2C_6H_4(2-F)$ | $CH_3$ |
| $C-CH_3$ | N | $C-C_2H_5$ | O | $CH_2C_6H_4(4-F)$ | $CH_3$ |
| $C-CH_3$ | N | $C-C_2H_5$ | O | $CH_2C_6H_3(2-Cl,6-F)$ | $CH_3$ |
| $C-CH_3$ | N | $C-C_2H_5$ | O | $CH_2C_6H_4(2-CH_3)$ | $CH_3$ |
| $C-CH_3$ | N | $C-C_2H_5$ | O | $CH_2C_6H_4(4-OCH_3)$ | $CH_3$ |
| $C-CH_3$ | N | $C-C_2H_5$ | O | $CH_2C_6H_2(3,4,5-tri-OCH_3)$ | $CH_3$ |
| $C-CH_3$ | N | $C-C_2H_5$ | O | $CH_2C_6H_4(4-OCH_2-C_6H_5)$ | $CH_3$ |
| $C-CH_3$ | N | $C-C_2H_5$ | O | $C_2H_5$ | $(CH_2)_3CON(CH_3)C_6H11$ |
| $C-CH_3$ | N | $C-C_2H_5$ | O | $CH_3$ | $CH_2C_6H_4(4-F)$ |
| $C-CH_3$ | N | $C-C_2H_5$ | O | $C_2H_5$ | $CH_2C_6H_4(4-F)$ |
| $C-CH_3$ | N | $C-C_2H_5$ | O | $CH_2C_6H_2(3,4,5-tri-OCH_3)$ | $CH_2C_6H_2(3,4,5-tri-OCH_3)$ |
| $C-CH_3$ | N | $C-C_2H_5$ | O | $C_2H_5$ | 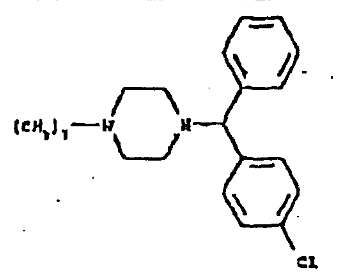 |

**2.** Process for producing pyrido[3,2-e]pyrazinones of formula I, wherein A, X, Y, Z, $R^1$,$R^2$ $R^3$, $R^4$ and $R^5$ have the meaning given in claim 1, **characterised in that** compounds of formula

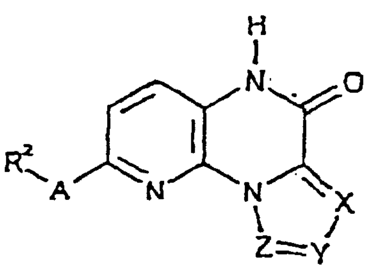

II

wherein X, Y, Z, A and $R^2$ have the meaning given in claim 1, are reacted with $R^1$-Hal (Hal = halogen) in the presence of an inorganic or organic basic catalyst, wherein $R^1$ has the meaning given in claim 1.

**3.** Process for producing pyrido[3,2-e]pyrazinones of formula I, wherein A, X, Y, Z, $R^1$, $R^2$, $R^3$, $R^4$ and $R^5$ have the meaning given in claim 1, **characterised in that** compounds of formula

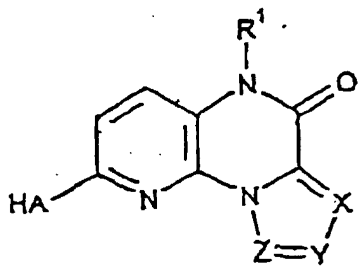

III

wherein A, X, Y, Z and $R^1$ have the meaning given in claim 1, are reacted with $R^2$-Hal (Hal = halogen) in the presence of an inorganic or organic basic catalyst, wherein $R^2$ has the meaning given in claim 1.

**4.** Process according to any one or more of the preceding claims, **characterised in that** basic compounds of formula I are converted into salts.

**5.** Process according to any one or more of the preceding claims, **characterised in that** acidic compounds of formula I are converted into salts.

**6.** Compounds of formula I according to claim 1 for use as therapeutic agents with anti-asthmatic and anti-allergic activity.

**7.** Pharmaceutical composition containing one or more compounds of formula I according to claim 1 in addition to conventional physiologically compatible excipients and/or diluents or auxiliary agents.

**8.** Process for producing a pharmaceutical composition according to claim 7, **characterised in that** one or more compound(s) of formula I are processed with conventional pharmaceutical excipients and/or diluents or other auxiliary agents to form pharmaceutical preparations or are brought into a therapeutically usable form.

**9.** Use of compounds of general formula I according to claim 1 for producing pharmaceutical compositions according to claims 7 and 8 to treat asthmatic and allergic conditions alone or in combination with one another or in combination with carriers and/or diluents or other auxiliary agents.

**Revendications**

**1.** Pyrido[3,2-e]pyrazinones de formule

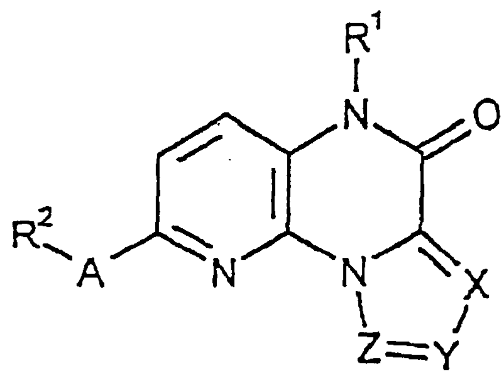

I

dans laquelle

A représente un atome d'oxygène;

X, Y et Z représentent N ou $CR^3$, au moins l'un des X, Y et Z devant être N;

$R^1$ représente un reste alkyle en $C_1$-$C_{10}$ (éventuellement ramifié), qui peut être substitué une ou plusieurs fois par des groupes hydroxy, alcoxy en $C_1$-$C_6$, alcényloxy ayant jusqu'à 6 atomes de carbone, alcynyloxy ayant jusqu'à 6 atomes de carbone, aryle, aryloxy, hétéroaryle, hétéroaryloxy, amino, halogéno, $NO_2$, CN, $C=OR^4$ ou $S(O)_nR^5$ (n allant de 0 à 2); un reste alcényle ayant jusqu'à 10 atomes de carbone (éventuellement ramifié), qui peut être substitué une ou plusieurs fois par des groupes hydroxy, alcoxy en $C_1$-$C_6$, alcényloxy ayant jusqu'à 6 atomes de carbone, alcynyloxy ayant jusqu'à 6 atomes de carbone, aryle, aryloxy, hétéroaryle, hétéroaryloxy, amino, halogéno, $NO_2$, CN, $C=OR^4$ ou $S(O)_nR^5$ (n allant de 0 à 2); ou un reste cycloalkyle en $C_5$-$C_7$ pouvant être substitué une ou plusieurs fois par des groupes hydroxy, alcoxy en $C_1$-$C_6$, alcényloxy ayant jusqu'à 6 atomes de carbone, alcynyloxy ayant jusqu'à 6 atomes de carbone, aryle, aryloxy, hétéroaryle, hétéroaryloxy, amino, halogéno, $NO_2$, CN, $C=OR^4$ ou $S(O)_nR^5$ (n allant de 0 à 2);

$R^2$ représente H; un reste alkyle en $C_1$-$C_{10}$ (éventuellement ramifié), qui peut être substitué une ou plusieurs fois par des groupes hydroxy, alcoxy en $C_1$-$C_6$, alcényloxy ayant jusqu'à 6 atomes de carbone, alcynyloxy ayant jusqu'à 6 atomes de carbone, aryle, aryloxy, hétéroaryle, hétéroaryloxy, amino, halogéno, $NO_2$, CN, $C=OR^4$ ou $S(O)_nR^5$ (n allant de 0 à 2); un reste alcényle ayant jusqu'à 10 atomes de carbone (éventuellement ramifié), qui peut être substitué une ou plusieurs fois par des groupes hydroxy, alcoxy en $C_1$-$C_6$, alcényloxy ayant jusqu'à 6 atomes de carbone, alcynyloxy ayant jusqu'à 6 atomes de carbone, aryle, aryloxy, hétéroaryle, hétéroaryloxy, amino, halogéno, $NO_2$, CN, $C=OR^4$ ou $S(O)_nR^5$ (n allant de 0 à 2); ou un reste cycloalkyle en $C_5$-$C_7$ pouvant être substitué une ou plusieurs fois par des groupes hydroxy, alcoxy en $C_1$-$C_6$, alcényloxy ayant jusqu'à 6 atomes de carbone, alcynyloxy ayant jusqu'à 6 atomes de carbone, aryle, aryloxy, hétéroaryle, hétéroaryloxy, amino, halogéno, $NO_2$, CN, $C=OR^4$ ou $S(O)_nR^5$ (n allant de 0 à 2);

$R^3$ représente H ou un reste alkyle en $C_1$-$C_6$ (éventuellement ramifié);

$R^4$ représente H ou un reste alkyle en $C_1$-$C_6$ (éventuellement ramifié); phényle; OH, alcoxy en $C_1$-$C_6$ (éventuellement ramifié); aryloxy; ou amino;

$R^5$ représente H ou un reste alkyle en $C_1$-$C_6$; aryle; OH; alcoxy en $C_1$-$C_6$; aryloxy; ou amino,

et leurs sels physiologiquement acceptables, ainsi que les composés dans lesquels

| X | Y | Z | A | $R^1$ | $R^2$ |
|---|---|---|---|---|---|
| $C-CH_3$ | N | $C-C_2H_5$ | O | $CH_2C_6H_4$(2-Cl) | $CH_3$ |
| $C-CH_3$ | N | $C-C_2H_5$ | O | $CH_2C_6H_4$(4-Cl) | $CH_3$ |
| $C-CH_3$ | N | $C-C_2H_5$ | O | $CH_2C_6H_3$(2,4-di-Cl) | $CH_3$ |
| $C-CH_3$ | N | $C-C_2H_5$ | O | $CH_2C_6H_3$(2,6-di-Cl) | $CH_3$ |
| $C-CH_3$ | N | $C-C_2H_5$ | O | $CH_2C_6H_4$(2-F) | $CH_3$ |
| $C-CH_3$ | N | $C-C_2H_5$ | O | $CH_2C_6H_4$(4-F) | $CH_3$ |
| $C-CH_3$ | N | $C-C_2H_5$ | O | $CH_2C_6H_3$(2-Cl, 6-F) | $CH_3$ |
| $C-CH_3$ | N | $C-C_2H_5$ | O | $CH_2C_6H_4(2-CH_3)$ | $CH_3$ |
| $C-CH_3$ | N | $C-C_2H_5$ | O | $CH_2C_6H_4(4-OCH_3)$ | $CH_3$ |
| $C-CH_3$ | N | $C-C_2H_5$ | O | $CH_2C_6H_2(3,4,5\text{-tri-}OCH_3)$ | $CH_3$ |
| $C-CH_3$ | N | $C-C_2H_5$ | O | $CH_2C_6H_2(4-OCH_2-C_6H_5)$ | $CH_3$ |
| $C-CH_3$ | N | $C-C_2H_5$ | O | $C_2H_5$ | $(CH_2)_3CON(CH_3)C_6H_{11}$ |
| $C-CH_3$ | N | $C-C_2H_5$ | O | $CH_3$ | $CH_2C_6H_4$(4-F) |
| $C-CH_3$ | N | $C-C_2H_5$ | O | $C_2H_5$ | $CH_2C_6H_4$(4-F) |
| $C-CH_3$ | N | $C-C_2H_5$ | O | $CH_2C_6H_2(3,4,5\text{-tri-}OCH_3)$ | $CH_2C_6H_2(3,4,5\text{-tri-}OCH_3)$ |

| X | Y | Z | A | $R^1$ | $R^2$ |
|---|---|---|---|---|---|
| $C-CH_3$ | N | $C-C_2H_5$ | O | $C_2H_5$ | 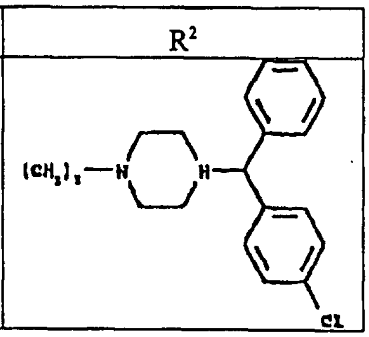 |

2. Procédé de préparation de pyrido[3,2-e]pyrazinones de formule I, dans laquelle A, X, Y, Z, $R^1$, $R^2$, $R^3$, $R^4$ et $R^5$ ont la signification indiquée dans la revendication 1, **caractérisé en ce que** l'on fait réagir des composés de formule

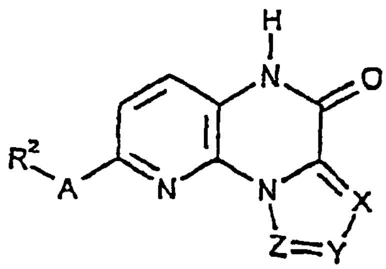

II

dans laquelle X, Y, Z, A et $R^2$ ont la signification indiquée dans la revendication 1, avec $R^1$-Hal (Hal = halogène) en présence d'un catalyseur basique inorganique ou organique, $R^1$ ayant la signification indiquée dans la revendication 1.

3. Procédé de préparation de pyrido[3,2-e]pyrazinones de formule I, dans laquelle A, X, Y, Z, $R^1$, $R^2$, $R^3$, $R^4$ et $R^5$ ont la signification indiquée dans la revendication 1, **caractérisé en ce que** l'on fait réagir des composés de formule

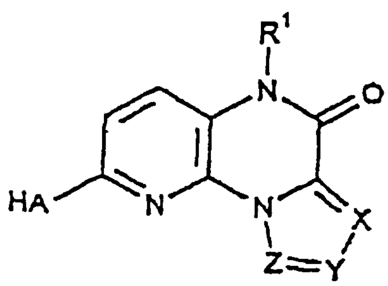

III

dans laquelle A, X, Y, Z et $R^1$ ont la signification indiquée dans la revendication 1, avec $R^2$-Hal (Hal = halogène) en présence d'un catalyseur basique inorganique ou organique, $R^2$ ayant la signification indiquée dans la revendication 1.

4. Procédé selon l'une ou plusieurs des revendications précédentes, **caractérisé en ce que** l'on transforme des composés basiques de formule I en sels.

5. Procédé selon l'une ou plusieurs des revendications précédentes, **caractérisé en ce que** l'on transforme des composés acides de formule I en sels.

6. Composés de formule I selon la revendication 1 pour l'utilisation comme substances actives thérapeutiques ayant des effets antiasthmatiques et antiallergiques.

**7.** Médicaments contenant un ou plusieurs composés de formule I selon la revendication 1 avec des supports et/ou des diluants ou des additifs physiologiquement acceptables classiques.

**8.** Procédé de préparation d'un médicament selon la revendication 7, **caractérisé en ce que** l'on transforme un ou plusieurs composés de formule I an compositions pharmaceutiques avec des supports et/ou des diluants ou d'autres additifs pharmaceutiques classiques, ou on les met sous une forme utilisable en thérapeutique.

**9.** Utilisation de composés de formule générale I selon la revendication 1 pour la préparation de médicament selon les revendications 7 et 8 à utiliser dans des maladies asthmatiques et allergiques, seuls ou en combinaison entre eux, ou en combinaison avec des supports et/ou des diluants ou d'autres additifs.